# EUROPEAN PATENT SPECIFICATION

(11) **EP 3 106 097 B1**
(45) Date of publication and mention of the grant of the patent: **28.11.2018**
(21) Application number: 15748965.9
(22) Date of filing: 05.02.2015
(51) Int. Cl.: A61B 17/00, A61B 17/29

(54) **TREATMENT INSTRUMENT**
BEHANDLUNGSINSTRUMENT
INSTRUMENT DE TRAITEMENT

(30) Priority: 12.02.2014 JP 2014024755
(43) Date of publication of application: 21.12.2016
(73) Proprietor: Olympus Corporation, Tokyo 192-8507 (JP)
(72) Inventor: TAKEI, Yusuke, Hachioji-shi Tokyo 192-8507 (JP)
(74) Representative: Schicker, Silvia
(86) International application number: PCT/JP2015/053268
(87) International publication number: WO 2015/122353

(56) References cited:
- EP-A1- 0 565 049
- EP-A1- 0 600 173
- EP-A1- 0 621 009
- EP-A1- 2 036 505
- JP-A- 2003 290 237
- JP-A- 2009 066 400
- JP-A- 2011 087 911
- JP-A- 2011 224 375
- US-A- 5 540 375
- US-A- 5 901 895

## Description

### Technical Field

This invention relates to a treatment device to treat living tissues.

### Background Art

For example, Jpn. Pat. Appln. KOKAI Publication No. 2012-35065 discloses a treatment device having an end effector which is rotatable relative to the distal end of a sheath. This treatment device locks and keeps the end effector rotated relative to the sheath so that a fixture may be easily attached to the end of the end effector. After the fixture is attached, the end effector is unlocked and thus held straight relative to the distal end of the sheath.

In a surgical treatment device, it may be necessary to temporarily hold the end effector rotated relative to the distal end of an insertion portion, for example, when water is continuously supplied or sucked. When the position of the end effector is temporarily held in this way, there is concern that the treatment device might be increased in size and deteriorate in operability if the number of parts needed for operations increases.

EP 2 036 505 A1 discloses a surgical stapler including an endoscopic portion with an elongated portion, an articulation portion, and an articulation joint located therebetween. The articulation joint is configured for manipulating a tool assembly mounted on a distal end of the articulation portion. An articulation rod extends from the articulation portion through the articulation joint into the elongated portion and terminates in a hook which engages with an articulation knob. The articulation knob is slidably mounted on the elongated portion. Proximal movement of the articulation knob causes the articulation rod to pull on the articulation joint, which in turn causes the articulation portion and the tool assembly to deflect at the articulation joint. Frictional engagement of the articulation knob with the elongated portion prevents the articulation joint from over articulating or straightening out.

EP 0 600 173 A1 describes an apparatus including an endoscopic section having, at a distal end portion, a staple storage section which pivots with respect to at least one side of a longitudinal axis extending centrally through the endoscopic section. A finger operable collar is mounted with the endoscopic section, the collar being slidable distally and proximally with respect to the endoscopic section. When the collar is moved distally or proximally, a pair of longitudinally extending push rods, which engage with proximal and distal links provided in the collar, are moved in equal and opposite directions, causing the staple storage section to pivot. The apparatus comprises a dual detent mechanism to positively establish positions of the staple storage section.

US 5,901,895 A discloses a surgical stapler including an elongate body portion and a fastener and cutting portion operatively associated with a distal end of the body portion. An articulation lever is pivotally mounted to a rotation handle. By pivoting the articulation lever, an actuation control link rod is caused to move proximally, which in turn articulates the fastener and cutting portion to one side of the surgical stapler and away from a central longitudinal axis thereof. The surgical stapler provides an articulation position maintaining arrangement which holds the fastener and cutting portion in one of three articulated positions.

EP 0 565 049 A1 discloses an endoscopic or laparoscopic surgical instrument comprising a handle assembly, a body assembly, a trigger and an internal locking mechanism assembly associated with the trigger. The body assembly comprises an outer tubular member through which an inner rod member coaxially passes in a slidable arrangement. Upon movement of a pivoting handle of the handle assembly, the inner rod reciprocates within the outer tube member to open and close jaws of a tool mechanism provided at a distal end of the body assembly. The locking mechanism assembly provides for retaining the tool mechanism in various positions by pulling on the trigger.

EP 0 621 009 A1 discloses a surgical instrument including an outer tube and an end effector portion capable of being angulated with respect to a longitudinal axis of the outer tube. Rotatory motion of an articulation knob assembly effects linear movement of an articulation tube provided in the outer tube, causing the end effector portion to angulate with respect to the longitudinal axis.

There are therefore demands for a treatment device in which the end effector can be temporarily held rotated relative to the distal end of the insertion portion by a simple operation.

### Summary of Invention

The present invention provides a treatment device according to independent claim 1. Preferred embodiments are defined by the dependent claims.

A treatment device in which an end effector can be temporarily held rotated relative to the distal end of an insertion portion by a simple operation is described.

A treatment device may include an insertion portion which includes a distal end, a proximal end, and a longitudinal axis defined by the distal end and the proximal end; an end effector which is rotatably provided at the distal end of the insertion portion; a grasp portion which is provided at the proximal end of the insertion portion and which is grasped by a user; a transmitting member which moves back and forth along a longitudinal axis of the insertion portion to transmit a drive force to rotate the end effector; a first locking portion which is provided on the proximal side of the transmitting member; a second locking portion which is provided on the proximal side of the insertion portion and which is configured to engage with the first locking portion; and an operational body which activates the first locking portion relative to the second locking portion, which is configured to switch between a first position where the transmitting member is moved forward along the longitudinal axis of the insertion portion to keep the end effector in a first state to extend along the longitudinal axis relative to the distal end of the insertion portion along the longitudinal axis, and a
second position where the transmitting member is moved backward along the longitudinal axis of the insertion portion to keep the end effector in a second state to be rotated relative to the longitudinal axis with respect to the distal end of the insertion portion.

### Brief Description of Drawings

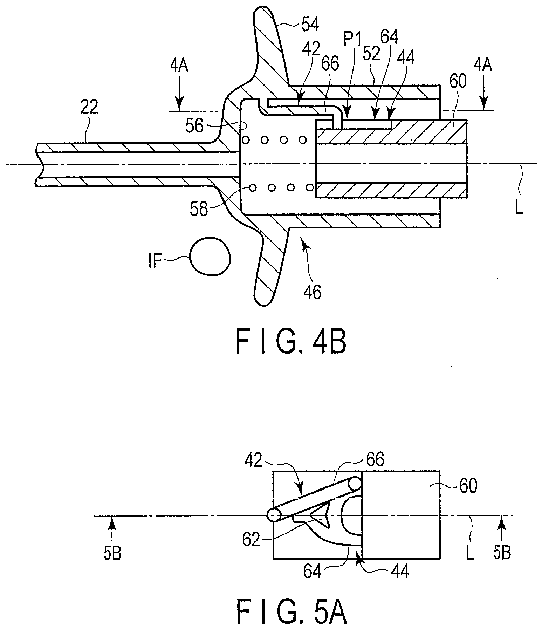

FIG. 1A is a schematic partly sectional view showing a treatment device according to an embodiment and an example useful for understanding the invention;
FIG. 1B is a schematic diagram showing a position holding mechanism to hold the position of a transmitting member relative to a grasp portion of the treatment device according to the embodiment and the example useful for understanding the invention;
FIG. 1C is a schematic diagram showing the position holding mechanism to hold the position of the transmitting member relative to the grasp portion of the treatment device according to the embodiment and the example useful for understanding the invention;
FIG. 2 is a schematic partly sectional perspective view showing the position holding mechanism to hold the position of the transmitting member relative to the grasp portion of the treatment device according to the embodiment and the example useful for understanding the invention;
FIG. 3A is a schematic diagram showing a first locking portion which is disposed on the proximal side of the transmitting member in relation to a second locking portion which is disposed in the grasp portion of the treatment device according to the embodiment;
FIG. 3B is a schematic sectional view taken along the
   line 3B-3B in FIG. 3A, showing the first locking portion which is disposed on the proximal side of the transmitting member in relation to the second locking portion which is disposed in the grasp portion of the treatment device according to the embodiment;
FIG. 4A is a schematic partly sectional view taken along the line 4A-4A in FIG. 4B, showing the position of an engagement pawl of the first locking portion which is disposed on the proximal side of the transmitting member in relation to an engagement groove of the second locking portion which is disposed in the grasp portion of the treatment device according to the embodiment;
FIG. 4B is a schematic sectional view taken along the line 4B-4B in FIG. 4A, showing the position of the engagement pawl of the first locking portion which is disposed on the proximal side of the transmitting member in relation to the engagement groove of the second locking portion which is disposed in the grasp portion of the treatment device according to the embodiment;
FIG. 5A is a schematic partly sectional view taken along the line 5A-5A in FIG. 5B, showing the position of the engagement pawl of the first locking portion which is disposed on the proximal side of the transmitting member in relation to the engagement groove of the second locking portion which is disposed in the grasp portion of the treatment device according to the embodiment;
FIG. 5B is a schematic sectional view taken along the
   line 5B-5B in FIG. 5A, showing the position of the engagement pawl of the first locking portion which is disposed on the proximal side of the transmitting member in relation to the engagement groove of the second locking portion which is disposed in the grasp portion of the treatment device according to the embodiment;

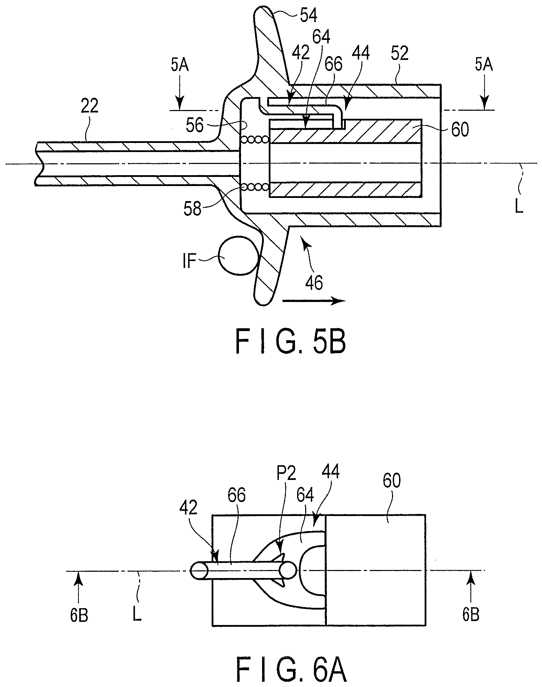

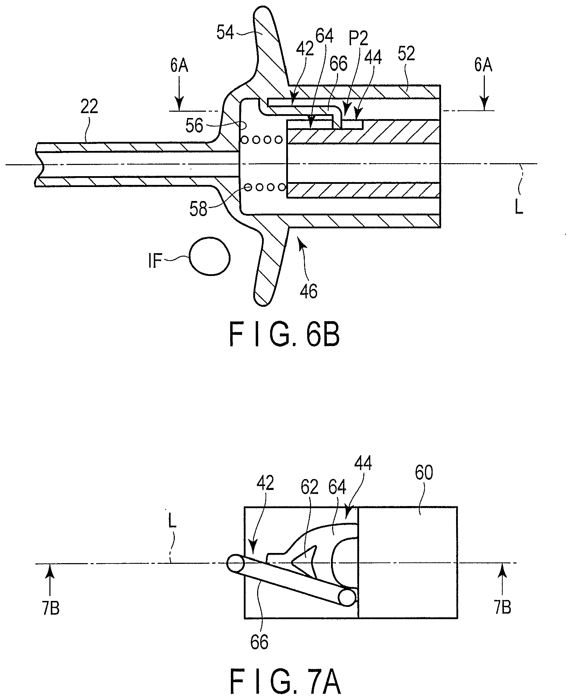

FIG. 6A is a schematic partly sectional view taken along the line 6A-6A in FIG. 6B, showing the position of the engagement pawl of the first locking portion which is disposed on the proximal side of the transmitting member in relation to the engagement groove of the second locking portion which is disposed in the grasp portion of the treatment device according to the embodiment;
FIG. 6B is a schematic sectional view taken along the line 6B-6B in FIG. 6A, showing the position of the engagement pawl of the first locking portion which is disposed on the proximal side of the transmitting member in relation to the engagement groove of the second locking portion which is disposed in the grasp portion of the treatment device according to the embodiment;

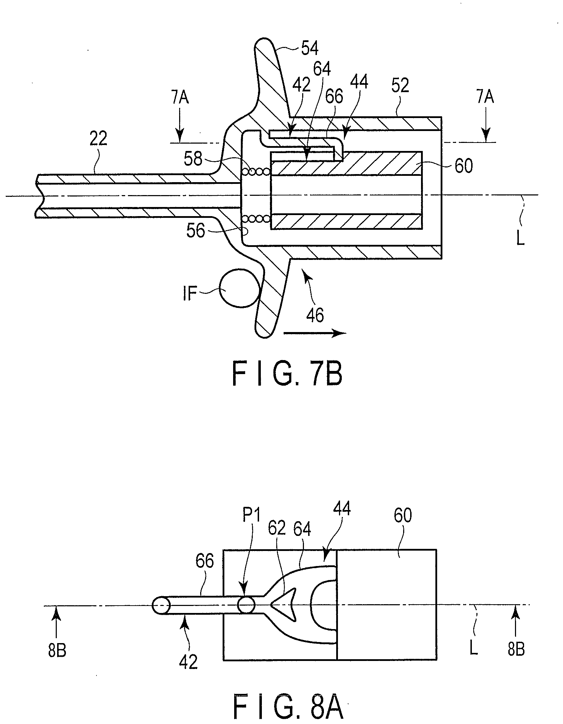

FIG. 7A is a schematic partly sectional view taken along the line 7A-7A in FIG. 7B, showing the position of the engagement pawl of the first locking portion which is disposed on the proximal side of the transmitting member in relation to the engagement groove of the second locking portion which is disposed in the grasp portion of the treatment device according to the embodiment;
FIG. 7B is a schematic sectional view taken along the line 7B-7B in FIG. 7A, showing the position of the engagement pawl of the first locking portion which is disposed on the proximal side of the transmitting member in relation to the engagement groove of the second locking portion which is disposed in the grasp portion of the treatment device according to the embodiment;

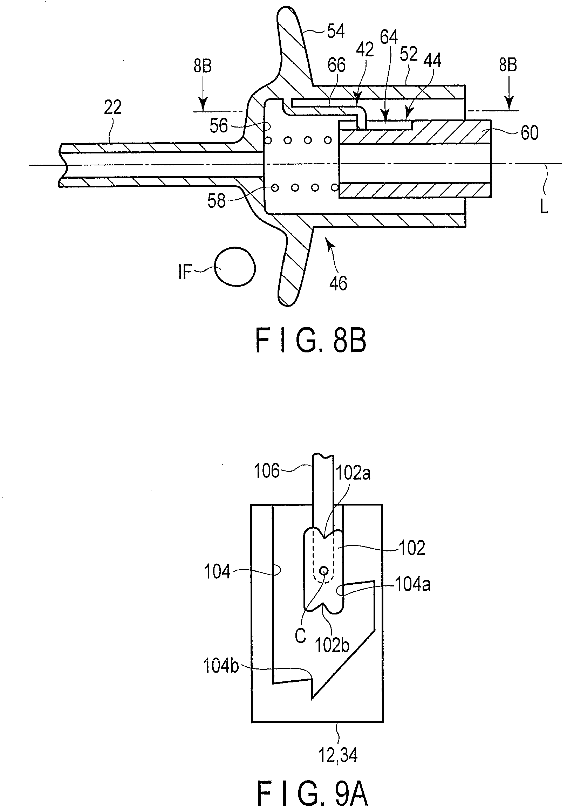

FIG. 8A is a schematic partly sectional view taken along the line 8A-8A in FIG. 8B, showing the position of the engagement pawl of the first locking portion which is disposed on the proximal side of the transmitting member in relation to the engagement groove of the second locking portion which is disposed in the grasp portion of the treatment device according to the embodiment;
FIG. 8B is a schematic sectional view taken along the line 8B-8B in FIG. 8A, showing the position of the engagement pawl of the first locking portion which is disposed on the proximal side of the transmitting member in relation to the engagement groove of the second locking portion which is disposed in the grasp portion of the treatment device according to the embodiment;
FIG. 9A is a schematic diagram showing a rotating cam type position holding mechanism of the treatment device according to the embodiment, and showing a state in which a cam provided in the transmitting member is disengaged from a recess formed in the outer circumferential surface of a grasp side cylindrical portion of the grasp portion;

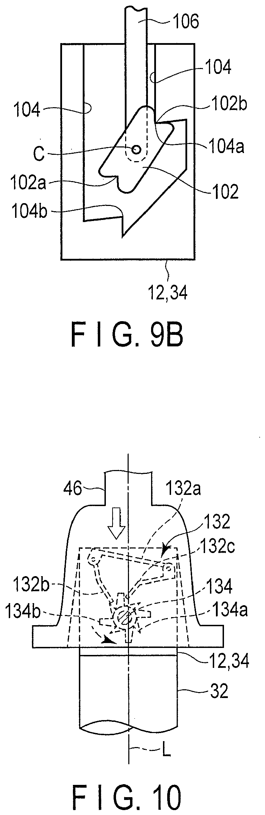

FIG. 9B is a schematic diagram showing the rotating cam type position holding mechanism of the treatment device according to the embodiment, and showing a state in which the cam provided in the transmitting member is engaged with the recess formed in the outer circumferential surface of the grasp side cylindrical portion of the grasp portion;
FIG. 10 is a schematic diagram showing a ratchet cam type position holding mechanism of the treatment device according to the embodiment, and showing a state in which the position of an operational body is moved relative to a pawl formed in the outer circumferential surface of the grasp side cylindrical portion of the grasp portion;

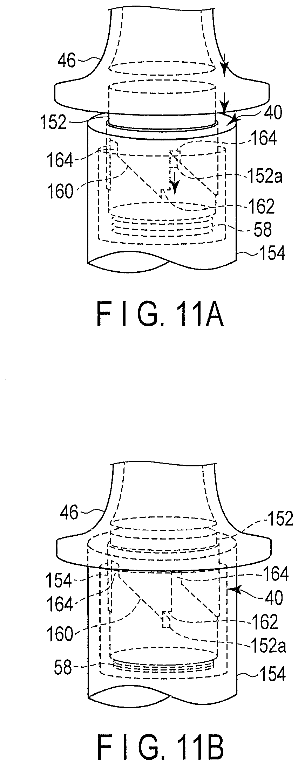

FIG. 11A is a schematic diagram showing the ratchet cam type position holding mechanism of the treatment device according to the embodiment, and showing a state in which a movable body and an elastic member are started to be pressed by the operational body from a state in which the movable body and the elastic member are supported on a tubular body;
FIG. 11B is a schematic diagram showing the ratchet cam type position holding mechanism of the treatment device according to the embodiment, and showing a state in which the movable body and the elastic member are pressed by the operational body so that a protrusion of the movable body is engaged with a first cutout in the inner
   circumferential surface of the tubular body;

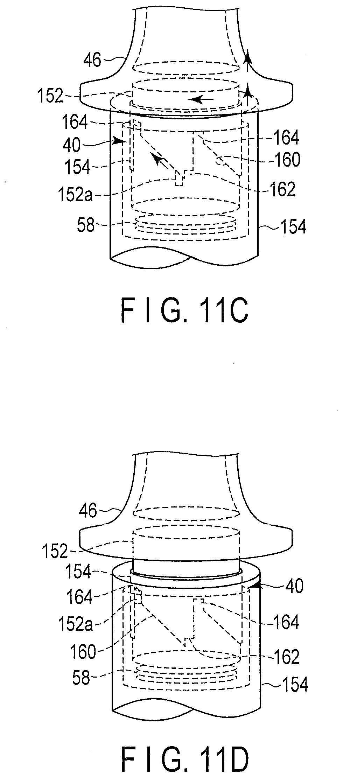

FIG. 11C is a schematic diagram showing the ratchet cam type position holding mechanism of the treatment device according to the embodiment, and showing a state in which the movable body and the elastic member are again pressed by the operational body so that the protrusion of the movable body is disengaged from the first cutout in the inner circumferential surface of the tubular body, and the protrusion is moving along an angle portion of the tubular body;
FIG. 11D is a schematic diagram showing the ratchet cam type position holding mechanism of the treatment device according to the embodiment, and showing a state in which the protrusion of the movable body is engaged with a second cutout of the tubular body;

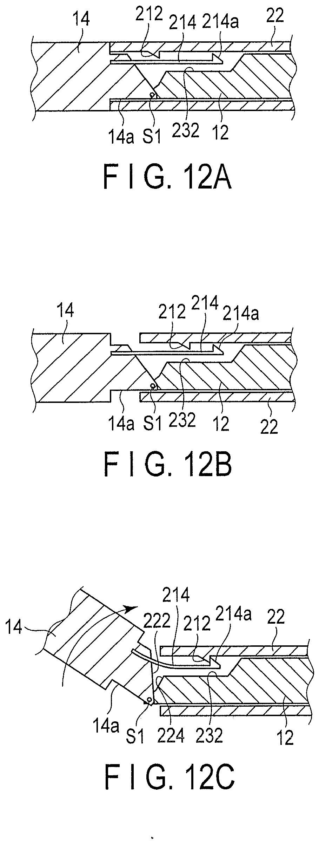

FIG. 12A is a schematic longitudinal sectional view showing a connection state between the proximal end of an end effector of the treatment device according to the example useful for understanding the invention and the sheath and the distal end of the transmitting member, and showing a state in which the end effector is straight along the longitudinal axis of the sheath;
FIG. 12B is a schematic longitudinal sectional view showing a connection state between the proximal end of the end effector of the treatment device according to the example useful for understanding the invention and the sheath and the distal end of the transmitting member, and showing a state in which the
   transmitting member has retreated relative to the sheath along the longitudinal axis;
FIG. 12C is a schematic longitudinal sectional view showing a connection state between the proximal end of the end effector of the treatment device according to the example useful for understanding the invention and the sheath and the distal end of the transmitting member, and showing a state in which the transmitting member retreats relative to the sheath along the longitudinal axis so that the end effector starts to rotate in a direction to deviate from the longitudinal axis;

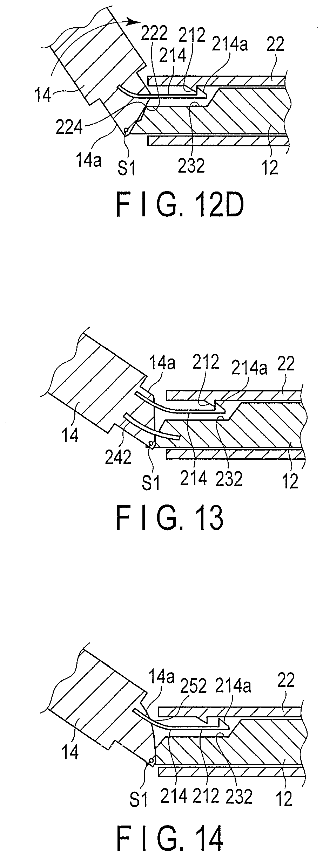

FIG. 12D is a schematic longitudinal sectional view showing a connection state between the proximal end of the end effector of the treatment device according to the example useful for understanding the invention and the sheath and the distal end of the transmitting member, and showing a state in which the transmitting member retreats relative to the sheath along the longitudinal axis so that the end effector is held rotated in the direction to deviate from the longitudinal axis;
FIG. 13 is a schematic longitudinal sectional view showing a connection state between the proximal end of the end effector of the treatment device according to a first modification of the example useful for understanding the invention and the sheath and the distal end of the transmitting member;
FIG. 14 is a schematic longitudinal sectional view showing a connection state between the proximal end of the
   end effector of the treatment device according to a second modification of the example useful for understanding the invention and the sheath and the distal end of the transmitting member; and

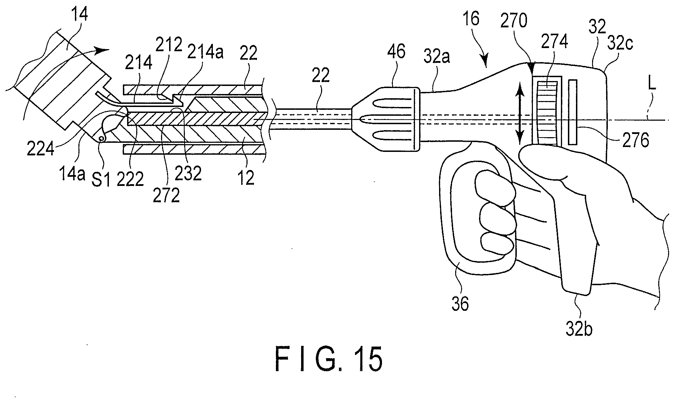

FIG. 15 is a schematic diagram showing a mechanism which adjusts the rotation amount of the end effector of the treatment device according to a third modification of the example useful for understanding the invention.

### Description of an Embodiment

Hereinafter, an embodiment of this invention will be described with reference to the drawings.

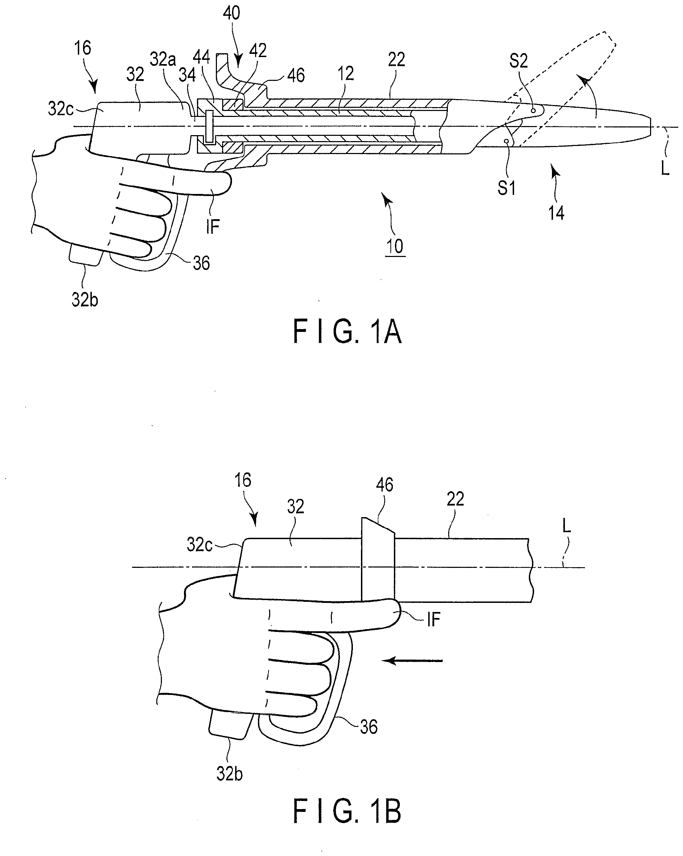

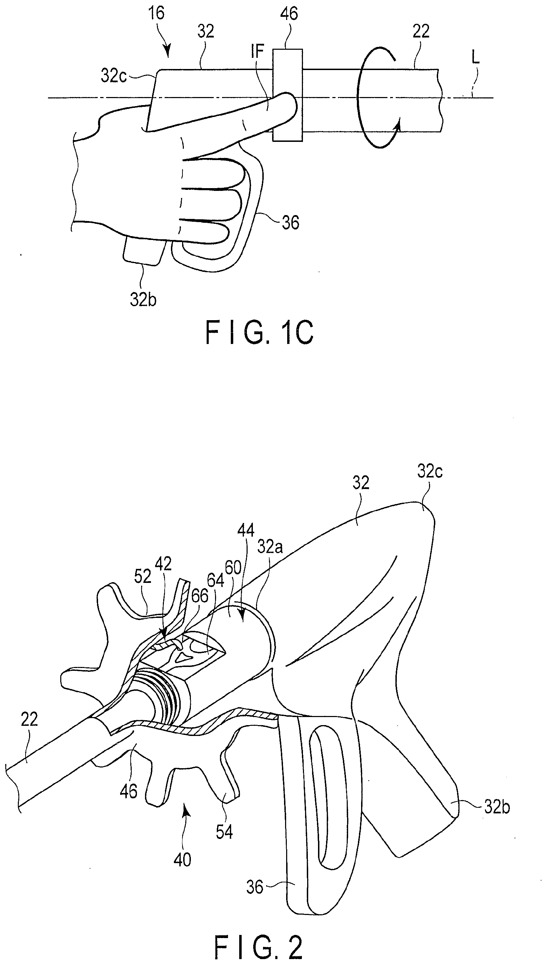

The embodiment is described with reference to FIG. 1A to FIG. 8B.

As shown in FIG. 1A, a treatment device 10 according to this embodiment has an elongated sheath (inner tube) 12, an end effector 14, and a grasp portion 16. The sheath 12 functions as an insertion portion to be inserted into, for example, a body cavity together with the end effector 14.

The sheath 12 has a distal end, a proximal end, and a longitudinal axis (central axis) L defined by the distal end and the proximal end. Thus, a front (distal side) and a back (proximal side) are defined along the longitudinal axis L. The sheath 12 is cylindrically formed in this embodiment. The sheath 12 is formed as an inner tube which is disposed inside a later-described transmitting member 22 as an outer tube. Thus, the longitudinal axis L is also the central axis of the sheath 12 and the transmitting member 22. The proximal end of the end effector 14 is coupled to the distal end of the sheath 12 rotatably on a first rotation shaft S1. The first rotation shaft S1 intersects at right angles with the longitudinal axis (central axis) L, and is located apart from the longitudinal axis (central axis) L.

The end effector 14 is, for example, openable and closable by a known mechanism. Thus, the end effector 14 can hold and push away living tissues. The end effector 14 is rotatably provided at the distal end of the sheath 12.

The transmitting member (outer tube) 22 which can move back and forth along the longitudinal axis L and which can transmit a drive force to rotate the end effector 14 to the distal end of the sheath 12 is disposed in the sheath 12. The transmitting member 22 is cylindrically formed as an outer tube outside the sheath 12 in this embodiment. The distal end of the transmitting member 22 is coupled so that the proximal end of the end effector 14 is rotatable on a second rotation shaft S2.

The end effector 14 according to this embodiment is formed to be rotatable only in one direction from a straight state. The second rotation shaft S2 is located apart from the longitudinal axis (central axis) L and is parallel to the first rotation shaft S1. That is, the second rotation shaft S2 intersects at right angles with the longitudinal axis (central axis) L. The second rotation shaft S2 is located opposite to the first rotation shaft S1 across the longitudinal axis (central axis) L.

If the transmitting member 22 is moved forward relative to the sheath 12 along the longitudinal axis L, the end effector 14 is disposed at a position (first position) where the end effector 14 is straight along the longitudinal axis L. If the transmitting member 22 is moved backward relative to the sheath 12 along the longitudinal axis L, the end effector 14 is disposed at a position (second position) where the end effector 14 is rotated to a predetermined rotation angle relative to the longitudinal axis L.

As shown in FIG. 1A to FIG. 1C, the grasp portion 16 is provided at the proximal end of the sheath 12, and is grasped by a user. The grasp portion 16 has a main body 32 which is substantially L-shaped like a pistol to be grasped in this embodiment, a grasp side cylindrical portion 34, and an open-close knob 36.

The main body 32 has one end 32a, the other end 32b, and a bent portion 32c. When grasping the grasp portion 16, the user of the treatment device 10 grasps a part ranging from the bent portion 32c to the other end 32b, and then extends, for example, the index to little fingers toward the one end 32a of the grasp portion 16.

The open-close knob 36 is disposed in the main body 32 of the grasp portion 16 when the end effector 14 has a pair of openable and closable jaws (not shown). The open-close knob 36 is disposed from the bent portion 32c to the one end 32a of the main body 32. The open-close knob 36 can move closer to or away from the other end 32b of the main body 32. The open-close knob 36 is coupled to the end effector 14 by, for example, an unshown wire which is inserted through the sheath 12. For example, when the open-close knob 36 is moved closer to the other end 32b of the main body 32, the wire is pulled so that the pair of jaws are relatively closed. For example, when the open-close knob 36 is moved away from the other end 32b of the main body 32, the wire is pushed out so that the pair of jaws are relatively opened. It is possible to relatively open and close the pair of jaws by operating the open-close knob 36 in this way.

The grasp side cylindrical portion 34 is disposed at the one end 32a of the main body 32. The proximal end of the sheath 12 is connected rotatably relative to the grasp side cylindrical portion 34 of the grasp portion 16 around the longitudinal axis L. A later-described base 60 of a second locking portion 44 is also rotatable relative to the grasp side cylindrical portion 34 around the longitudinal axis L.

As shown in FIG. 1A and FIG. 2 to FIG. 8B, a first locking portion 42 is provided on the proximal side of the transmitting member 22. The second locking portion 44 which can engage with the first locking portion 42 is provided in the outer circumferential surface of the grasp side cylindrical portion 34 of the grasp portion 16 or in the outer circumferential surface of the proximal end of the sheath 12. That is, the second locking portion 44 which can engage with the first locking portion 42 is provided on the proximal side of the sheath 12. In the explanation according to this embodiment, the sheath 12 and the transmitting member 22 rotate around the longitudinal axis L, so that the second locking portion 44 is provided in the outer circumferential surface of the proximal end of the sheath 12. If the sheath 12 and the transmitting member 22 do not need to rotate around the longitudinal axis L, the second locking portion 44 may be provided in the outer circumferential surface of the grasp side cylindrical portion 34 of the grasp portion 16. An operational body 46 which activates the first locking portion 42 relative to the second locking portion 44 is formed on the proximal side of the transmitting member 22. The first locking portion 42, the second locking portion 44, and the operational body 46 form a position holding mechanism (alternate mechanism) 40 by which the first locking portion 42 and the second locking portion 44 hold positions relative to each other. The position holding mechanism 40 is provided between the transmitting member 22 and the grasp portion 16.

As shown in FIG. 2 and FIG. 4A to FIG. 8B, the operational body 46 is integrated with the proximal end of the transmitting member 22 in this embodiment. The operational body 46 has a cylindrical portion 52, and a substantially disc-shaped extension 54 which is disposed on the outer circumference of the cylindrical portion 52 and which extends outward in the diametrical direction.

The cylindrical portion 52 is formed so that the later-described base 60 of the second locking portion 44 is disposed inside the cylindrical portion 52.

As described above, the extension 54 is located so that the user of the treatment device 10 can move the extension 54 along the longitudinal axis L by putting an index finger IF on the extension 54 when the user grasps the part of the grasp portion 16 ranging from the bent portion 32c to the other end 32b and then extends the index to little fingers toward the one end 32a of the grasp portion 16. Thus, the index finger IF is put on the extension 54 to move the transmitting member 22 along the direction of the longitudinal axis L. A step 56 is formed in the operational body 46 between the operational body 46 and the proximal end of the transmitting member 22, and the distal end of a later-described urging member 58 is supported by the step 56.

The urging member 58, for example, a coil spring is disposed between the step 56 of the operational body 46 and the distal end of the later-described cylindrical base 60. The urging member 58 urges the transmitting member 22 forward along the direction of the longitudinal axis L.

As shown in FIG. 2 to FIG. 8B, the position holding mechanism 40 is formed as a heart cam type in this embodiment.

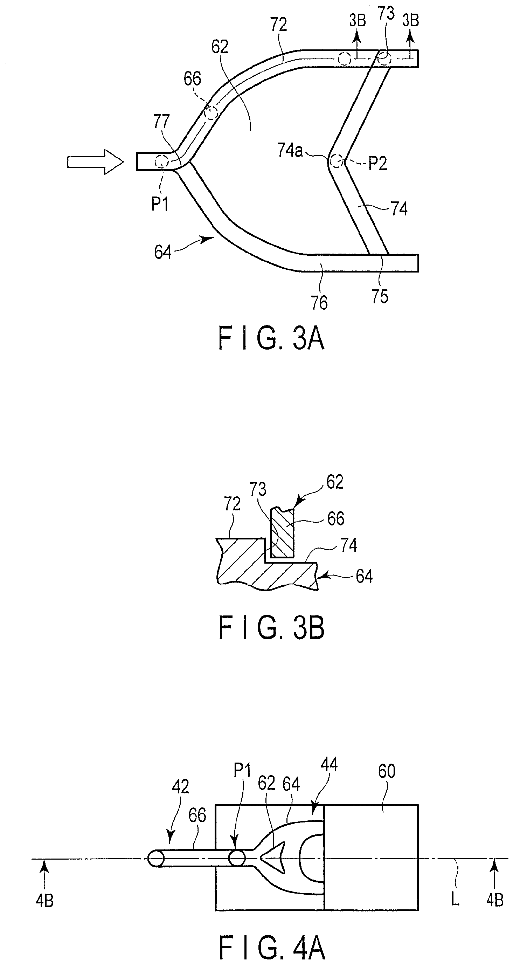

As shown in FIG. 3A and FIG. 3B, the second locking portion 44 has the cylindrical base 60, a heart-shaped island 62 formed in the outer circumferential surface of the base 60, and a locking groove 64 formed around the island 62. The first locking portion 42 has an engagement pawl 66 which is movable along the locking groove 64 and which is elastically deformable. The engagement pawl 66 is urged to be movable along the bottom surface of the locking groove 64. The engagement pawl 66 is movable between an unlock position (first position) P1 and a lock position (second position) P2 of the locking groove 64 that will be described later, and is held at the unlock position P1 and the lock position P2. That is, if the engagement pawl 66 of the first locking portion 42 traces the locking groove 64 of the second locking portion 44, the engagement pawl 66 moves from the unlock position P1 to the lock position P2 of the locking groove 64, or from the lock position P2 to the unlock position P1. The engagement pawl 66 of the position holding mechanism 40 then performs an alternating action along the locking groove 64 by the urging member 58.

The locking groove 64 has a first groove portion 72, a second groove portion 74, and a third groove portion 76. The locking groove 64 forms the aforementioned island 62 inside by the first to third groove portions 72, 74, and 76. The boundary between the first groove portion 72 and the second groove portion 74 has a step 73 in which the first groove portion 72 is formed high and the second groove portion 74 is formed low. The boundary between the second groove portion 74 and the third groove portion 76 has a step 75 in which the second groove portion 74 is formed high and the third groove portion 76 is formed low. The boundary between the third groove portion 76 and the first groove portion 72 has a step 77 in which the third groove portion 76 is formed high and the first groove portion 72 is formed low.

The first groove portion 72 is formed higher in the vicinity of the boundary between the first groove portion 72 and the second groove portion 74 than in the vicinity of the boundary between the first groove portion 72 and the third groove portion 76. The second groove portion 74 is formed lower in the vicinity of the boundary between the second groove portion 74 and the first groove portion 72 than in the vicinity of the boundary between the second groove portion 74 and the third groove portion 76. The third groove portion 76 is formed higher in the vicinity of the boundary between the third groove portion 76 and the first groove portion 72 than in the vicinity of the boundary between the third groove portion 76 and the second groove portion 74. That is, each of the first groove portion 72 to the third groove portion 76 is formed as an inclined surface.

The first groove portion 72 has the unlock position (first position) P1 where the engagement pawl 66 is disengaged in the vicinity of the boundary between the first groove portion 72 and the third groove portion 76. The second groove portion 74 is substantially V-shaped, that is, has a bent portion 74a, and the bent portion 74a functions as the lock position (second position) P2 of the engagement pawl 66.

At the unlock position (first position) P1, the transmitting member 22 is moved forward along the longitudinal axis L of the sheath 12 by the operational body 46. That is, the transmitting member 22 is moved to a front position. In this instance, the end effector 14 is kept straight along the longitudinal axis L relative to the distal end of the sheath 12. At the lock position (second position) P2, the transmitting member 22 is moved backward along the longitudinal axis L of the sheath 12 by the operational body 46. That is, the transmitting member 22 is moved backward and then slightly returned forward and is thereby moved to a back position located backward of the front position. In this instance, the end effector 14 is kept rotated relative to the longitudinal axis L with respect to the distal end of the sheath 12. These positions are switched by the operation of the operational body 46. That is, the operational body 46 of the position holding mechanism 40 can be switched between the unlock position (first position) P1 where the transmitting member 22 is kept relatively moved forward along the longitudinal axis L to keep the end effector 14 in a first state to be straight along the longitudinal axis L, and the lock position (second position) P2 where the transmitting member 22 is kept relatively moved backward along the longitudinal axis L to keep the end effector 14 in a second state to be rotated relative to the longitudinal axis L at the distal end of the sheath 12. The position holding mechanism 40 is switched between the first position and the second position by the alternating action.

Functions according to this embodiment are described with reference to FIG. 1A to FIG. 1c and FIG. 4A to FIG. 8B.

As shown in FIG. 1A, the user grasps the position of the grasp portion 16 ranging from the bent portion 32c to the other end 32b and then extends the index finger IF toward the operational body 46 to put the index finger IF on the extension 54. The user inserts the end effector 14, the distal end of the sheath 12, and the distal end of the transmitting member 22 into, for example, a body cavity.

As shown in FIG. 4A and FIG. 4B, in a state in which the operational body 46 is relatively moved forward along the longitudinal axis L to keep the end effector 14 straight along the longitudinal axis L, the engagement pawl 66 of the first locking portion 42 is disposed at the position (unlock position P1) of the first groove portion 72 in the boundary between the first groove portion 72 and the third groove portion 76 of the second locking portion 44.

From this state, the extension 54 of the operational body 46 is operated backward on the longitudinal axis L. That is, the first locking portion 42 is moved relative to the second locking portion 44. The engagement pawl 66 of the first locking portion 42 cannot move from the first groove portion 72 of the second locking portion 44 to the third groove portion 76 due to the step 77, and moves toward the boundary between the first groove portion 72 and the second groove portion 74 from the unlock position P1 as shown in FIG. 5A and FIG. 5B.

In this instance, the transmitting member 22 is pulled backward by a component in a direction along the longitudinal axis L in the distance between the unlock position P1 and the boundary between the first groove portion 72 and the second groove portion 74. That is, the transmitting member 22 is pulled backward relative to the sheath 12. Thus, the end effector 14 is pulled on the second rotation shaft S2, and the end effector 14 rotates around the first rotation shaft S1. In this instance, the living tissues can be, for example, pushed away.

When the engagement pawl 66 reaches the second groove portion 74 from the first groove portion 72, direct return of the engagement pawl 66 to the first groove portion 72 from the second groove portion 74 is regulated by the step 73. As shown in FIG. 6A to FIG. 6C, the engagement pawl 66 is disposed at the lock position P2 by the urging force of the urging member 58 when the operational body 46 is released.

At this point, the distance between the unlock position P1 and the lock position P2 is smaller than a component in the direction along the longitudinal axis L in the distance between the unlock position P1 of the transmitting member 22 and the boundary between the first groove portion 72 and the second groove portion 74. Thus, the rotation amount of the end effector 14 is smaller than the maximum rotation amount state. Since the position of the engagement pawl 66 is regulated, the end effector 14 maintains a certain amount of rotation relative to the distal end of the sheath 12. Thus, this treatment device 10 allows the end effector 14 to be temporarily held rotated relative to the distal end of the sheath 12 by a simple operation.

When the end effector 14 has a pair of openable and closable jaws, the pair of jaws can be relatively opened and closed by an operation to move the open-close knob 36 disposed in the grasp portion 16 closer to or away from the other end 32b of the grasp portion 16. At this point, it is possible to keep grasping the living tissue while the end effector 14 is rotated relative to the distal end of the sheath 12.

When the end effector 14 is located at a position where the end effector 14 is bent relative to the distal end of the sheath 12, the end effector 14 shakes if an external force is applied to the end effector 14. However, unless the extension 54 is moved backward along the longitudinal axis L, a large rotation that may cause the end effector 14 to be straight relative to the sheath 12 is prevented. Since the sheath 12 and the transmitting member 22 have concentric cylindrical shapes in this embodiment, the sheath 12 and the transmitting member 22 are low in flexibility and can maintain high rigidity.

If the extension 54 is rotated around the longitudinal axis L, the transmitting member 22 and the sheath 12 rotate relative to the grasp portion 16 because the engagement pawl 66 of the first locking portion 42 is engaged with the locking groove 64 of the second locking portion 44. Thus, it is possible to suitably adjust the direction of the end effector 14 relative to the grasp portion 16. The living tissue can be easily treated by such a suitable adjustment of the direction of the end effector 14.

After the living tissue is grasped and suitably treated with the end effector 14, the distal end of the sheath 12 and the end effector 14 are pulled out of the body cavity. Before this work, the living tissue is released. Further work is done so that the end effector 14 becomes straight relative to the distal end of the sheath 12 along the longitudinal axis L. The extension 54 is operated backward along the longitudinal axis L while the engagement pawl 66 of the first locking portion 42 is disposed in the second groove portion 74 (located at the lock position P2). As shown in FIG. 7A and FIG. 7B, the engagement pawl 66 moves toward the boundary between the second groove portion 74 and the third groove portion 76 from the lock position P2. When the first locking portion 42 reaches the third groove portion 76 from the second groove portion 74, direct return of the engagement pawl 66 to the second groove portion 74 from the third groove portion 76 is regulated by the step 75.

The boundary between the second groove portion 74 and the third groove portion 76 is located backward as compared to the lock position P2 along the longitudinal axis L. The transmitting member 22 is pulled backward relative to the sheath 12. Thus, the rotation amount of the end effector 14 temporarily surpasses the lock position P2.

If the extension 54 of the operational body 46 is released, the engagement pawl 66 is disposed at the unlock position P1 of the first groove portion 72 by the urging force of the urging member 58. In this instance, direct return of the engagement pawl 66 to the third groove portion 76 from the first groove portion 72 is regulated by the step 77. Accordingly, the engagement pawl 66 moves to the position of the boundary between the first groove portion 72 and the third groove portion 76 from the position of the boundary between the second groove portion 74 and the third groove portion 76. Therefore, as shown in FIG. 8A and FIG. 8B, the transmitting member 22 moves forward relative to the sheath 12. Thus, the end effector 14 is pushed out on the second rotation shaft S2, and the end effector 14 rotates around the first rotation shaft S1. Consequently, the end effector 14 becomes straight relative to the distal end of the sheath 12 along the longitudinal axis L. In this state, the distal end of the sheath 12 and the end effector 14 are pulled out of the body cavity.

As described above, the following can be said according to the treatment device 10 in this embodiment.

The user can easily switch the end effector 14 at the distal end of the sheath 12 to the position to be straight along the longitudinal axis L and the position to be bent simply by grasping the grasp portion 16 of the treatment device 10 with one hand and then operating the extension 54 of the operational body 46 backward along the longitudinal axis L of the sheath 12 to release the extension 54. Thus, according to the treatment device 10 in this embodiment, it is possible to temporarily hold the end effector 14 rotated relative to the distal end of the sheath 12 by a simple operation.

In this instance, for example, the extension 54 of the operational body 46 is only operated backward along the longitudinal axis L with the index finger while the open-close knob 36 is being held with one of the middle to little fingers. If the extension 54 is released after the extension 54 of the operational body 46 has been operated backward along the longitudinal axis L with the index finger, the transmitting member 22 can be switched by the urging force of the urging member 58 between the position (unlock position) P1 where the transmitting member 22 is moved forward relative to the sheath 12 and the position (lock position) P2 where the transmitting member 22 is moved backward. That is, the treatment device 10 according to this embodiment allows the end effector 14 at the distal end of the sheath 12 to be switched to a suitable position solely by one-hand operation.

The extension 54 of the operational body 46 can be rotated around the longitudinal axis L with the index finger. By this operation, the sheath 12, the end effector 14, and the transmitting member 22 can be suitably rotated around the longitudinal axis L. This allows the end effector 14 to face in a suitable direction. Performing this operation also only requires one hand.

In the example described according to this embodiment, the position holding mechanism 40 using the heart-shaped cam type is used. Instead of using the heart-shaped cam type, the position holding mechanism 40 may use, for example, a rotating cam type shown in FIG. 9A and FIG. 9B, a ratchet cam type shown in FIG. 10, or a rotary cam type shown in FIG. 11A to FIG. 11D.

The position holding mechanism 40 shown in FIG. 9A and FIG. 9B has a cam (first locking portion) 102 which moves along the longitudinal axis L, and a recessed frame (second locking portion) 104 which contacts the circumferential end face of the cam 102.

For example, the cam 102 has a moving rod 106 which moves parallel to the longitudinal axis L in accordance with the motion of the operational body 46. The recessed frame 104 is disposed in the outer circumferential surface of the grasp side cylindrical portion 34 of the grasp portion 16, and is rotatable around the longitudinal axis L, but the movement of the recessed frame 104 in the forward and backward direction is preferably regulated.

The cam 102 has cutouts 102a and 102b at both ends, and has a central shaft C rotatably attached to the moving rod 106. The recessed frame 104 has two separately disposed corners, 104a and 104b, with which the cutouts 102a and 102b engage.

The point of contact between the cam 102 and the recessed frame 104 moves in accordance with the backward movement of the operational body 46, that is, the backward movement of the moving rod 106. The cam 102 holds the active state when the distal-side corner 104a of the two corners 104a and 104b of the recessed frame 104 enters one of the cutouts 102a and 102b of the rotating cam 102. That is, the lock position is set.

If the operational body 46, that is, the moving rod 106 is again moved backward, the corner 104a of the recessed frame 104 comes off one of the cutouts 102a and 102b, and the cam 102 rotates in contact with the proximal-side corner 104b. If the operational force is then removed, the cam 102 returns to a free position as shown in FIG. 9A. That is, the unlock position is set.

Therefore, the user can temporarily hold the end effector 14 rotated relative to the distal end of the sheath (insertion portion) 12 by a simple operation of the operational body 46.

As shown in FIG. 10, the position holding mechanism 40 has a pawl (first locking portion) 132 having both its ends supported, and a cam (second locking portion) 134. The pawl 132 is supported on the operational body 46, and the cam 134 is disposed, for example, in the outer circumferential surface of the grasp side cylindrical portion 34 of the grasp portion 16 or in the outer circumferential surface of the proximal end of the sheath 12. The pawl 132 has an input portion 132a which is activated by the operational body 46, a feed pawl 132b which rotates the cam 134 together with the input portion 132a, and a stop pawl 132c which stops the rotation of the cam 134 that has been rotated by a predetermined amount together with the input portion 132a. The cam 134 has a feed pawl 134a which is rotated a predetermined amount by the feed pawl 132b, and a stop pawl 134b which stops the rotation of the cam 134 that has been rotated by a predetermined amount by the stop pawl 132c.

The operational force is transmitted to the feed pawl 132b via the input portion 132a by the operational body 46 located at the front position along the longitudinal axis L shown in FIG. 10. The cam 134 rotates in a direction indicated by an arrow in FIG. 10. However, one operation of the operational body 46 alone does not rotate the cam 134 by a certain amount or more due to the stop pawl 132c. If the operational body 46 is again operated to push the feed pawl 132b, the cam 134 again rotates. Thus, the operational body 46 is movable within a predetermined range along the longitudinal axis L. This operation is repeated so that the operational body 46 is switched between the unlock position and the lock position. Therefore, the end effector 14 moves as shown in FIG. 1A.

Therefore, the user can temporarily hold the end effector 14 rotated relative to the distal end of the sheath (insertion portion) 12 by a simple operation of the operational body 46.

As shown in FIG. 11A to FIG. 11D, the position holding mechanism (alternate mechanism) 40 has a movable body (first locking portion) 152 having, for example, a cylindrical shape, and a tubular body (second locking portion) 154 disposed on the outer circumference of the movable body 152. The movable body 152 is provided on the proximal side of the transmitting member 22. The tubular body (second locking portion) 154 is provided in the outer circumferential surface of the grasp side cylindrical portion 34 or in the outer circumferential surface of the proximal end of the sheath 12.

The movable body 152 is urged upward (toward the end effector 14) in FIG. 11A to FIG. 11D by the urging member 58. The movable body 152 has, in its outer circumferential surface, a protrusion 152a. The tubular body 154 has, in its inner circumferential surface, cutouts 162 and 164 in a back end side portion and a root portion (distal end side portion) of a saw-tooth-shaped angle portion 160.

In FIG. 11A, the operational body 46 is at the unlock position (the position where the end effector 14 extends along the longitudinal axis L of the sheath 12). As shown in FIG. 11A, the movable body 152 is urged upward in FIG. 11A by the urging force of the urging member 58, and the protrusion 152a of the movable body 152 engages with the second cutout 164 formed in the root portion of the angle portion 160 in the inner circumferential surface of the tubular body 154. That is, the movable body 152 is pushed upward (closer to the end effector 14) by the urging force of the urging member 58 applied to the inner circumferential surface (bottom surface) of the tubular body 154, and the protrusion 152a of the movable body 152 engages with the second cutout 164 formed in the root portion of the angle portion 160 of the tubular body 154.

If the operational body 46 located at the position shown in FIG. 11A is then pushed toward the back end along the longitudinal axis L, the movable body 152 is pushed by the operational body 46 and thus contracts the urging member 58. If the force to push the operational body 46 is loosened in this state, the movable body 152 is slightly lifted upward by the urging force of the urging member 58. In this instance, the protrusion 152a of the movable body 152 engages with the first cutout 162 formed in the distal portion of the angle portion 160 of the tubular body 154. Thus, the movable body 152 shown in FIG. 11B is kept moved toward the back end as compared to the movable body 152 shown in FIG. 11A.

If the operational body 46 in the state shown in FIG. 11B is again pushed, the movable body 152 contracts the urging member 58 and is then slightly pushed down, so that the protrusion 152a of the movable body 152 which has engaged with the first cutout 162 of the tubular body 154 comes off the first cutout 162. If the force to push the operational body 46 is loosened in this state, the movable body 152 is lifted upward by the urging force of the urging member 58. Accordingly, the movable body 152 moves relative to the tubular body 154 from the state shown in FIG. 11C to the state shown in FIG. 11D.

Therefore, the user can temporarily hold the end effector 14 rotated relative to the distal end of the sheath (insertion portion) 12 by a simple operation of the operational body 46.

Moreover, the movable body (first locking portion) 152 rotates relative to the tubular body 154. In this instance, an unshown support such as a ball bearing is disposed between the operational body 46 and the movable body 152, and prevents the transmitting member 22 from rotating when the movable body 152 rotates relative to the tubular body 154. The operational body 46 and the transmitting member 22 are supported by a suitable frictional force to prevent the operational body 46 and the transmitting member 22 from being automatically rotated by, for example, gravitation. The transmitting member 22 is rotated around the longitudinal axis L by the operation of the operational body 46.

Next, the example useful for understanding the invention is described with reference to FIG. 12A to FIG. 12D. This example is a modification of the embodiment, and the same components or the components having the same functions as those described in the embodiment are provided with the same reference marks and are not described in detail.

The structure described in this example increases the rigidity to keep the end effector 14 straight when the end effector 14 is straight relative to the distal end of the sheath 12.

The user can temporarily hold the end effector 14 rotated relative to the distal end of the sheath (insertion portion) 12 by a simple operation of the operational body 46.

In this example, instead of the second rotation shaft S2, a first locking body 212 is disposed in the inner circumferential surface of the distal end of the transmitting member 22, and a second locking body 214 is disposed in the end effector 14. The first locking body 212 is formed as an engagement protrusion in the inner circumferential surface of the distal end of the transmitting member 22. The second locking body 214 is
formed as a belt-shaped body having, at its proximal end, an engagement protrusion 214a which is switched between a state to be caught on the first locking body 212 (caught state) and a state to be released from the caught state (non-caught state). The second locking body 214 has a proper level of flexibility. The second locking body 214 may be suitably expandable in its longitudinal direction.

As shown in FIG. 12A to FIG. 12D, a recess (fit portion) 14a into which the distal end of the transmitting member (outer tube) 22 is fitted is formed at the proximal end of the end effector 14. The recess (fit portion) 14a is formed by removing the outer circumference of the proximal end of the end effector 14 in a tubular shape so that the distal end (fit portion) of the transmitting member 22 is fitted in an abutment state as shown in FIG. 12A.

Owing to the position holding mechanism 40, the distal end (fit portion) of the transmitting member 22 is fitted into the recess (fit portion) 14a at the proximal end of the end effector 14 as shown in FIG. 12A when the transmitting member 22 is located forward relative to the sheath 12. Thus, when the transmitting member 22 is located forward relative to the sheath 12, the end effector 14 is straight along the longitudinal axis L.

In contrast, when the transmitting member 22 is located backward relative to the sheath 12, the distal end of the transmitting member 22 is unfitted from the recess 14a at the proximal end of the end effector 14 as shown in FIG. 12B to FIG. 12D. Thus, the second locking body 214 is caught on the first locking body 212, and the end effector 14 is rotated to deviate from the longitudinal axis L.

The first locking body 212 and the second locking body 214 are engaged and disengaged when the engagement pawl 66 of the position holding mechanism 40 moves backward further than the lock position P2 along the longitudinal axis L from the unlock position P1 and then moves forward.

The proximal end of the end effector 14 has a first abutment surface (end effector side abutment surface) 222 which abuts on the distal end of the sheath 12. The distal end of the sheath 12 has a second abutment surface (sheath side abutment surface) 224 which abuts on the first abutment surface 222 at the proximal end of the end effector 14. If the first abutment surface 222 and the second abutment surface 224 are formed into suitable shapes and their abutment positions are adjusted, the rotation angle of the end effector 14 to the distal end of the sheath 12 can be adjusted.

When the end effector 14 is straight relative to the distal end of the sheath 12, the recess 14a at the proximal end of the end effector 14 is disposed inside the distal end of the transmitting member 22. Thus, when an external force is applied to the end effector 14 from the direction that deviates from the longitudinal axis L, the rotation of the end effector 14 relative to the distal end of the sheath 12 can be inhibited by the distal end of the transmitting member 22.

When the end effector 14 is straight relative to the distal end of the sheath 12, the first locking body 212 is located closer to the proximal side than the proximal end of the end effector 14 while the recess 14a at the proximal end of the end effector 14 is disposed inside the distal end of the transmitting member 22. Thus, it is possible to prevent the first locking body 212 from interfering with the end effector 14.

In contrast, when the end effector 14 is bent relative to the distal end of the sheath 12, the distal end of the transmitting member 22 moves backward relative to the distal end of the sheath 12 to expose the circular recess 14a at the proximal end of the end effector 14.

A guide portion 232 which moves the first locking body 212 and the second locking body 214 of the transmitting member 22 are provided at the distal end of the sheath 12. This guide portion 232 prevents the second locking body 214 from interfering with the distal end of the sheath 12 and the inner circumferential surface of the distal end of the transmitting member 22 when the transmitting member 22 is moved as shown in FIG. 12A to FIG. 12D.

Next, a first modification of the example useful for understanding the invention is described with reference to FIG. 13. This modification is a modification of the example useful for understanding the invention, and the same components or components having the same functions as those described in the embodiment and example useful for understanding the invention are provided
with the same reference marks and are not described in detail.

The user can temporarily hold the end effector 14 rotated relative to the distal end of the sheath (insertion portion) 12 by a simple operation of the operational body 46.

As shown in FIG. 13, an urging body 242 which urges the end effector 14 to rotate relative to the distal end of the sheath 12 is disposed between the distal end of the sheath 12 and the proximal end of the end effector 14. Thus, the end effector 14 can be easily rotated relative to the distal end of the sheath 12 by the urging force of the urging body 242 in addition to the tensile force of the second locking body 214. In the meantime, while the recess 14a at the proximal end of the end effector 14 is fitted in the inner circumferential surface of the distal end of the cylindrical transmitting member 22, the end effector 14 is kept straight relative to the distal end of the sheath 12. This prevents the state in which the end effector 14 is kept straight relative to the distal end of the sheath 12 from being affected even if the urging body 242 extends.

Next, a second modification of the example useful for understanding the invention is described with reference to FIG. 14. This modification is a modification of the example useful for understanding the invention, and the same components or components having the same functions as those described in the embodiment and the example useful for understanding the invention including the first modification are provided with the same
reference marks and are not described in detail.

The user can temporarily hold the end effector 14 rotated relative to the distal end of the sheath (insertion portion) 12 by a simple operation of the operational body 46.

A taper portion 252 in which the first abutment surface 222 is tapered is provided. Thus, the inner circumferential surface of the distal end of the transmitting member 22 can be easily guided to the recess 14a of the end effector 14 by the taper portion 252, and the end effector 14 can be easily moved when the end effector 14 is disposed straight along the longitudinal axis L relative to the distal end of the sheath 12.

Next, a third modification of the example useful for understanding the invention is described with reference to FIG. 15. This modification is a modification of the first and second modifications of the example useful for understanding the invention, and the same components or components having the same functions as those described in the embodiment and the example useful for understanding the invention including the first and second modifications are provided with the same reference marks and are not described in detail.

The user can temporarily hold the end effector 14 rotated relative to the distal end of the sheath (insertion portion) 12 by a simple operation of the operational body 46.

In this modification, an adjustment mechanism 270 which adjusts the amount of rotation of the end effector 14
relative to the distal end of the sheath 12 is described. The adjustment mechanism 270 can adjust a rotation angle at which the end effector 14 around the first rotation shaft S1 is kept in the second state where the end effector 14 is rotated relative to the longitudinal axis L at the distal end of the sheath 12.

The first abutment surface 222 at the proximal end of the end effector 14 is preferably disposed on the longitudinal axis (central axis) L either in the state in which the end effector 14 is straight or in the state in which the end effector 14 is rotated relative to the distal end of the sheath 12.

In this example useful for understanding the invention, an abutment rod (moving body) 272 which is movable in the direction of the longitudinal axis (central axis) L is inserted on the longitudinal axis L inside the sheath 12. The distal end of the abutment rod 272 can be in and out of contact with the first abutment surface 222. The proximal end of the abutment rod 272 is coupled to an adjustment knob (rotation amount adjustment body) 274 disposed in the grasp portion 16 via an unshown ball screw. The adjustment knob 274 is disposed in the grasp portion 16 rotatably around the longitudinal direction of the abutment rod 272. The grasp portion 16 exposes part of the adjustment knob 274 to the outside. The abutment rod 272 and the adjustment knob 274 constitute the adjustment mechanism 270.

An index 276 indicating the maximum rotation amount of
the end effector 14 is preferably disposed in the vicinity of the adjustment knob 274 of the main body 32 of the grasp portion 16.

If the adjustment knob 274 is suitably rotated, the abutment rod 272 moves forward and backward along the longitudinal axis L owing to the effects of the ball screw. The adjustment knob 274 moves the abutment rod 272 along its axial direction and adjusts the position where the abutment rod 272 abuts on the end effector 14 to adjust the amount of rotation of the end effector 14 relative to the distal end of the sheath 12. The maximum rotation amount is reduced if the abutment rod 272 is moved forward. The maximum rotation amount is increased if the abutment rod 272 is moved backward. At this point, it is possible to check the maximum rotation amount of the end effector 14 relative to the longitudinal axis L of the sheath 12, for example, solely by visual recognition.

### Reference Signs List

10: treatment device, 12: sheath (insertion portion), 14: end effector, 16: grasp portion, 22: transmitting member, 32: grasp portion main body, 34: grasp side cylindrical portion, 36: open-close knob, 40: position
holding mechanism (alternate mechanism), 42: first locking portion, 44: second locking portion, 46: operational body, 52: cylindrical portion, 54: extension, 56: step, 58: urging member, 60: base, 62: island, 64: locking groove, 66: locking pawl, 72: first groove portion, 73: step, 74: second groove portion, 75: step, 76: third groove portion, 77: step.

## Claims

1. A treatment device (10) comprising:
an insertion portion (12) which includes a distal end, a proximal end, and a longitudinal axis (L) defined by the distal end and the proximal end;
an end effector (14) which is coupled to the distal end of the insertion portion rotatably on a first rotation shaft (S1), wherein said first rotation shaft (S1) intersects at a right angle with the longitudinal axis (L) and is located apart from the longitudinal axis (L);
a transmitting member (22) which is coupled to the end effector (14) so that the end effector (14) is rotatable on a second rotation shaft (S2) and transmits a drive force of the transmitting member to the end effector to rotate the end effector, wherein said second rotation shaft (S2) is parallel to the first rotation shaft (S1) and is located opposite to the first rotation shaft (S1) across the longitudinal axis (L),
wherein if the transmitting member (22) is moved forward relative to the insertion portion (12) along the longitudinal axis (L), the end effector (14) is disposed at a first position, and if the transmitting member (22) is moved backward relative to the insertion portion (12) along the longitudinal axis (L), the end effector (14) is disposed at a second position;
a first locking portion (42; 102; 132; 152) which is provided on the proximal side of the transmitting member;
a second locking portion (44; 104; 134; 154) which is provided on the proximal side of the insertion portion and which is configured to engage with the first locking portion; and
an operational body (46) which is provided on the proximal side of the transmitting member (22), which activates the first locking portion (42) relative to the second locking portion (44), which is configured to switch between a first position where the transmitting member (22) is moved forward along the longitudinal axis (L) of the insertion portion (12) to keep the end effector (14) in a first state to extend along the longitudinal axis (L) relative to the distal end of the insertion portion (12) along the longitudinal axis (L), and a second position where the transmitting member (22) is moved backward along the longitudinal axis (L) of the insertion portion (12) to keep the end effector (14) in a second state to be rotated relative to the longitudinal axis (L) with respect to the distal end of the insertion portion (12);
**characterized by** further comprising
an urging body (58) which is disposed between the second locking portion (44) and the operational body (46) and urges the operational body forward along the longitudinal axis (L) of the insertion portion (12) or which is disposed between the second locking portion (44) and the transmitting member (22) and urges the transmitting member (22) forward along the longitudinal axis (L) of the insertion portion (12).

2. The treatment device (10) according to claim 1, wherein the first and second locking portions (42, 44) and the operational body (46) form a position holding mechanism (40) by which the first and second locking portions (42, 44) hold positions relative to each other.

3. The treatment device (10) according to claim 2, wherein the first and second locking portions (42, 44) of the position holding mechanism (46) are one of a heart cam type, a rotating cam type, a ratchet cam type, and a rotary cam type.

4. The treatment device (10) according to claim 1, wherein the first locking portion (42), the second locking portion (44), and the operational body (46) are rotatable around the longitudinal axis (L).

5. The treatment device (10) according to claim 1, wherein
the insertion portion (12) is formed as an inner tube, and
the transmitting member (22) is formed as an outer tube which is movable relative to the inner tube along the longitudinal axis (L).

6. The treatment device (10) according to claim 5, wherein
the insertion portion (12) includes the first rotation shaft (S1) which intersects at right angles with the longitudinal axis (L) and which serves as a supporting point of the rotation of the end effector (14), and
the operational body (46) includes an adjustment mechanism (270) configured to adjust a rotation angle at which the end effector (14) around the first rotation shaft (S1) is rotated relative to the longitudinal axis (L) at the distal end of the insertion portion (12) and kept in the second state.

7. The treatment device (10) according to claim 6, comprising a grasp portion (16) provided at the proximal end of the insertion portion (12) and grasped by a user;
wherein:
the adjustment mechanism (270) includes:
a moving body (272) which is disposed parallel to the longitudinal axis (L) and which abuts on the end effector (14); and
a rotation amount adjustment body (274) which is provided in the grasp portion (16) and which moves the moving body (272) along its axial direction and adjusts the position where the moving body (272) abuts on the end effector (14) to adjust the amount of rotation of the end effector (14) relative to the distal end of the insertion portion (12).

8. The treatment device (10) according to claim 1, wherein the transmitting member (22) is cylindrical, and is movable between a front position where the transmitting member (22) moves forward relative to the insertion portion (12) along the longitudinal axis (L) and is fitted into the outer circumferential surface of the proximal end of the end effector (14) so that the end effector (14) is in a first state to extend along the longitudinal axis (L) relative to the distal end of the insertion portion (12), and a back position where the transmitting member (22) moves backward relative to the insertion portion (12) along the longitudinal axis (L) and is unfitted from the outer circumferential surface of the proximal end of the end effector (14) so that the end effector (14) is in the second state relative to the distal end of the insertion portion (12).

9. The treatment device (10) according to claim 8, wherein the end effector (14) includes a fit portion (14a) which is fitted into the distal end of the transmitting member (22) in the first state.

## Patentansprüche

1. Behandlungsvorrichtung (10), umfassend:
einen Einsetzabschnitt (12), der ein distales Ende, ein proximales Ende und eine Längsachse (L) umfasst, die von dem distalen Ende und dem proximalen Ende definiert wird;
einen Endeffektor (14), der mit dem distalen Ende des Einsetzabschnitts an einer ersten Drehwelle (S1) drehbar gekoppelt ist, wobei die erste Drehwelle (S1) die Längsachse (L) in einem rechten Winkel schneidet und sich abseits der Längsachse (L) befindet;
ein Übertragungselement (22), das derart mit dem Endeffektor (14) gekoppelt ist, dass der Endeffektor (14) an einer zweiten Drehwelle (S2) gedreht werden und eine Antriebskraft des Übertragungselements an den Endeffektor übertragen kann, um den Endeffektor zu drehen,
wobei die zweite Drehwelle (S2) parallel zu der ersten Drehwelle (S1) ist und sich gegenüber der ersten Drehwelle (S1) über der Längsachse (L) befindet, wobei, wenn das Übertragungselement (22) bezogen auf den Einsetzabschnitt (12) entlang der Längsachse (L) vorwärtsbewegt wird, der Endeffektor (14) in einer ersten Position angeordnet ist, und wenn das Übertragungselement (22) bezogen auf den Einsetzabschnitt (12) entlang der Längsachse (L) rückwärts bewegt wird, der Endeffektor (14) in einer zweiten Position angeordnet ist;
einen ersten Sperrabschnitt (42; 102; 132; 152), der auf der proximalen Seite des Übertragungselements vorgesehen ist;
einen zweiten Sperrabschnitt (44; 104; 134; 154), der auf der proximalen Seite des Einsetzabschnitts vorgesehen und dazu ausgelegt ist, in den ersten Sperrabschnitt einzugreifen; und
einen Bedienungskörper (46), der auf der proximalen Seite des Übertragungselements (22) vorgesehen ist, der den ersten Sperrabschnitt (42) bezogen auf den zweiten Sperrabschnitt (44) aktiviert, der dazu ausgelegt ist, zwischen einer ersten Position zu schalten, in der das Übertragungselement (22) entlang der Längsachse (L) des Einsetzabschnitts (12) vorwärtsbewegt wird, um den Endeffektor (14) in einem ersten Zustand zu halten, um sich entlang der Längsachse (L) bezogen auf das distale Ende des Einsetzabschnitts (12) entlang der Längsachse (L) zu erstrecken, und einer zweiten Position, in der das Übertragungselement (22) entlang der Längsachse (L) des Einsetzabschnitts (12) rückwärtsbewegt wird, um den Endeffektor in einem zweiten Zustand zu halten, um bezogen auf die Längsachse (L) mit Bezug auf das distale Ende des Einsetzabschnitts (12) gedreht zu werden;
**gekennzeichnet dadurch, dass** sie ferner umfasst
einen Drängkörper (58), der zwischen dem zweiten Sperrabschnitt (44) und dem Bedienungskörper (46) angeordnet ist und den Bedienungskörper entlang der Längsachse (L) des Einsetzabschnitts (12) vorwärtsdrängt, oder der zwischen dem zweiten Sperrabschnitt (44) und dem Übertragungselement (22) angeordnet ist und das Übertragungselement (22) entlang der Längsachse (L) des Einsetzabschnitts (12) vorwärtsdrängt.

2. Behandlungsvorrichtung (10) nach Anspruch 1, wobei der erste und der zweite Sperrabschnitt (42, 44) und der Bedienungskörper (46) einen Positionshaltemechanismus (40) bilden, durch den der erste und der zweite Sperrabschnitt (42, 44) die Positionen bezogen aufeinander halten.

3. Behandlungsvorrichtung (10) nach Anspruch 2, wobei der erste und der zweite Sperrabschnitt (42, 44) des Positionshaltemechanismus (46) von einem herzförmigen Nockentyp, einem rotierenden Nockentyp, einem Ratschennockentyp oder einem Drehnockentyp sind.

4. Behandlungsvorrichtung (10) nach Anspruch 1, wobei der erste Sperrabschnitt (42), der zweite Sperrabschnitt (44) und der Bedienungskörper (46) um die Längsachse (L) drehbar sind.

5. Behandlungsvorrichtung (10) nach Anspruch 1, wobei
der Einsetzabschnitt (12) als ein Innenrohr gebildet ist, und
das Übertragungselement (22) als ein Außenrohr gebildet ist, das bezogen auf das Innenrohr entlang der Längsachse (L) beweglich ist.

6. Behandlungsvorrichtung (10) nach Anspruch 5, wobei
der Einsetzabschnitt (12) die erste Drehwelle (S1) umfasst, die die Längsachse (L) in rechten Winkeln schneidet, und die als ein Stützpunkt der Drehung des Endeffektors (14) dient, und
der Bedienungskörper (46) einen Einstellungsmechanismus (270) umfasst, der dazu ausgelegt ist, einen Drehwinkel einzustellen, in dem der Endeffektor (14) um die erste Drehwelle (S1) bezogen auf die Längsachse (L) an dem distalen Ende des Einsetzabschnitts (12) gedreht und in dem zweiten Zustand gehalten wird.

7. Behandlungsvorrichtung (10) nach Anspruch 6, umfassend einen Greifabschnitt (16), der an dem proximalen Ende des Einsetzabschnitts (12) vorgesehen ist und von einem Nutzer gegriffen wird;
wobei:
der Einstellungsmechanismus (270) umfasst:
einen Bewegungskörper (272), der parallel zu der Längsachse (L) angeordnet ist und
der an den Endeffektor (14) grenzt; und
einen Drehbetrageinstellungskörper (274), der in dem Greifabschnitt (16) vorgesehen ist und der den Bewegungskörper entlang seiner Achsrichtung bewegt und die Position einstellt, in der der Bewegungskörper (272) an den Endeffektor (14) grenzt, um den Betrag der Drehung des Endeffektors (14) bezogen auf das distale Ende des Einsetzabschnitts (12) einzustellen.

8. Behandlungsvorrichtung (10) nach Anspruch 1, wobei das Übertragungselement (22) zylindrisch ist und zwischen einer vorderen Position, in der das Übertragungselement bezogen auf den Einsetzabschnitt (12) entlang der Längsachse (L) vorwärtsbewegt und derart in die Außenumfangsfläche des proximalen Endes des Endeffektors (14) eingepasst wird, dass sich der Endeffektor (14) in einem ersten Zustand befindet, um sich entlang der Längsachse (L) bezogen auf das distale Ende des Einsetzabschnitts (12) zu erstrecken, und einer hinteren Position, in der sich das Sendelement (22) bezogen auf den Einsetzabschnitt (12) entlang der Längsachse (L) rückwärts bewegt und derart aus der Außenumfangsfläche des proximalen Endes des Endeffektors (14) herausbewegt wird, dass der Endeffektor (14) in dem zweiten Zustand bezogen auf das distale Ende des Einsetzabschnitts (12) ist, bewegbar ist.

9. Behandlungsvorrichtung (10) nach Anspruch 8, wobei der Endeffektor (14) einen Passabschnitt (14a) umfasst, der in das distale Ende des Übertragungselements (22) im ersten Zustand eingepasst wird.

## Revendications

1. Dispositif de traitement (10) comprenant :
une partie d'insertion (12) qui comprend une extrémité distale, une extrémité proximale et un axe longitudinal (L) défini par l'extrémité distale et l'extrémité proximale ;
un organe effecteur (14) qui est couplé à l'extrémité distale de la partie d'insertion de manière rotative sur un premier arbre de rotation (S1), ledit premier arbre de rotation (S1) coupant l'axe longitudinal (L) à angle droit et étant situé à distance de l'axe longitudinal (L) ;
un élément de transmission (22) qui est couplé à l'organe effecteur (14) de telle sorte que l'organe effecteur (14) est apte à tourner sur un second arbre de rotation (S2) et transmet une force d'entraînement de l'élément de transmission à l'organe effecteur pour faire tourner l'organe effecteur, ledit second arbre de rotation (S2) étant parallèle au premier arbre de rotation (S1) et étant situé opposé au premier arbre de rotation (S1) de l'autre côté de l'axe longitudinal (L), dans lequel, si l'élément de transmission (22) est déplacé vers l'avant par rapport à la partie d'insertion (12) le long de l'axe longitudinal (L), l'organe effecteur (14) est disposé dans une première position et, si l'élément de transmission (22) est déplacé vers l'arrière par rapport à la partie d'insertion (12) le long de l'axe longitudinal (L), l'organe effecteur (14) est disposé dans une seconde position ;
une première partie de verrouillage (42 ; 102 ; 132 ; 152) qui est prévue sur le côté proximal de l'élément de transmission ;
une seconde partie de verrouillage (44 ; 104 ; 134 ; 154) qui est prévue sur le côté proximal de la partie d'insertion et qui est configurée pour s'engager avec la première partie de verrouillage ; et
un corps fonctionnel (46) qui est prévu sur le côté proximal de l'élément de transmission (22), qui active la première partie de verrouillage (42) par rapport à la seconde partie de verrouillage (44), qui est configuré pour commuter entre une première position, dans laquelle l'élément de transmission (22) est déplacé vers l'avant le long de l'axe longitudinal (L) de la partie d'insertion (12) pour maintenir l'organe effecteur (14) dans un premier état pour s'étendre le long de l'axe longitudinal (L) par rapport à l'extrémité distale de la partie d'insertion (12) le long de l'axe longitudinal (L), et une seconde position dans laquelle l'élément de transmission (22) est déplacé vers l'arrière le long de l'axe longitudinal (L) de la partie d'insertion (12) pour maintenir l'organe effecteur (14) dans un second état pour être tourné par rapport à l'axe longitudinal (L) par rapport à l'extrémité distale de la partie d'insertion (12) ;
**caractérisé par le fait qu'**il comprend en outre un corps de poussée (58) qui est disposé entre la seconde partie de verrouillage (44) et le corps fonctionnel (46) et pousse le corps fonctionnel vers l'avant le long de l'axe longitudinal (L) de la partie d'insertion (12), ou qui est disposé entre la seconde partie de verrouillage (44) et l'élément de transmission (22) et pousse l'élément de transmission (22) vers l'avant le long de l'axe longitudinal (L) de la partie d'insertion (12).

2. Dispositif de traitement (10) selon la revendication 1, dans lequel les première et seconde parties de verrouillage (42, 44) et le corps fonctionnel (46) forment un mécanisme de maintien de position (40) par lequel les première et seconde parties de verrouillage (42, 44) maintiennent les positions l'une par rapport à l'autre.

3. Dispositif de traitement (10) selon la revendication 2, dans lequel les première et seconde parties de verrouillage (42, 44) du mécanisme de maintien de position (46) sont l'un parmi un type de came en coeur, un type de came tournante, un type de came de cliquet et un type de came rotative.

4. Dispositif de traitement (10) selon la revendication 1, dans lequel La première partie de verrouillage (42), la seconde partie de verrouillage (44) et le corps fonctionnel (46) sont aptes à tourner autour de l'axe longitudinal (L).

5. Dispositif de traitement (10) selon la revendication 1, dans lequel
la partie d'insertion (12) est sous la forme d'un tube interne, et
l'élément de transmission (22) est sous la forme d'un tube externe qui est mobile par rapport au tube interne le long de l'axe longitudinal (L).

6. Dispositif de traitement (10) selon la revendication 5, dans lequel
la partie d'insertion (12) comprend le premier arbre de rotation (S1) qui coupe l'axe longitudinal (L) à angles droits et qui sert de point de support de la rotation de l'organe effecteur (14), et
le corps fonctionnel (46) comprend un mécanisme d'ajustement (270) configuré pour ajuster un angle de rotation auquel l'organe effecteur (14) autour du premier arbre de rotation (S1) est tourné par rapport à l'axe longitudinal (L) au niveau de l'extrémité distale de la partie d'insertion (12) et maintenu dans le second état.

7. Dispositif de traitement (10) selon la revendication 6, comprenant une partie de préhension (16) prévue à l'extrémité proximale de la partie d'insertion (12) et saisie par un utilisateur ;
le mécanisme d'ajustement (270) comprenant :
un corps mobile (272) qui est disposé parallèle à l'axe longitudinal (L) et qui est en appui sur l'organe effecteur (14) ; et
un corps d'ajustement d'amplitude de rotation (274) qui est prévu dans la partie de préhension (16) et qui déplace le corps mobile (272) selon sa direction axiale et ajuste la position dans laquelle le corps mobile (272) est en appui sur l'organe effecteur (14) pour ajuster l'amplitude de rotation de l'organe effecteur (14) par rapport à l'extrémité distale de la partie d'insertion (12).

8. Dispositif de traitement (10) selon la revendication 1, dans lequel l'élément de transmission (22) est cylindrique et est mobile entre une position avant, dans laquelle l'élément de transmission (22) se déplace vers l'avant par rapport à la partie d'insertion (12) le long de l'axe longitudinal (L) et est monté dans la surface circonférentielle externe de l'extrémité proximale de l'organe effecteur (14) de telle sorte que l'organe effecteur (14) est dans un premier état pour s'étendre le long de l'axe longitudinal (L) par rapport à l'extrémité distale de la partie d'insertion (12), et une position arrière dans laquelle l'élément de transmission (22) se déplace vers l'arrière par rapport à la partie d'insertion (12) le long de l'axe longitudinal (L) et est démonté de la surface circonférentielle externe de l'extrémité proximale de l'organe effecteur (14) de telle sorte que l'organe effecteur (14) est dans le second état par rapport à l'extrémité distale de la partie d'insertion (12).

9. Dispositif de traitement (10) selon la revendication 8, dans lequel l'organe effecteur (14) comprend une partie de montage (14a) qui est montée dans l'extrémité distale de l'élément de transmission (22) dans le premier état.
